# EUROPEAN PATENT SPECIFICATION

(11) **EP 2 070 523 B1**
(45) Date of publication and mention of the grant of the patent: **18.08.2010**
(21) Application number: 08171088.1
(22) Date of filing: 09.12.2008
(51) Int. Cl.: A61K 31/05, A61K 36/575, A61P 29/02, A61P 19/02

(54) **Composition comprising Magnolol and Honokiol in synergistic amount**
Zusammensetzung enthaltend Magnolol und Honokiol in synergistischen Mengen
Composition comprenant Magnolol et Honokiol en quantités synergiques

(30) Priority: 11.12.2007 EP 07023983; 11.12.2007 EP 07023947
(43) Date of publication of application: 17.06.2009
(73) Proprietor: DSM IP Assets B.V., 6411 TE Heerlen (NL)
(72) Inventor: Kilpert, Claus, 68305, Mannheim (DE); Raederstorff, Daniel, 68720, Flaxlanden (FR); Richard, Nathalie, 68100, Mulhouse (FR); Schwager, Joseph, 4056, Basel (CH); Wertz, Karin, 79618, Rheinfelden (DE)
(74) Representative: Schwander, Kuno

(56) References cited:
- WO-A-2008/006581
- WO-A-2008/006582
- WO-A-2008/006589
- US-A1- 2006 074 108
- US-A1- 2006 140 885
- US-A1- 2006 216 251
- LEE JONGSUNG ET AL: "Anti-inflammatory effects of magnolol and honokiol are mediated through inhibition of the downstream pathway of MEKK-1 in NF-.kappa.B activation signaling" PLANTA MEDICA, THIEME, STUTTGART, DE, vol. 71, no. 4, 1 January 2005 (2005-01-01), pages 338-343, XP009088409 ISSN: 0032-0943
- LIN ET AL: "Effects of honokiol and magnolol on acute and inflammatory pain models in mice" LIFE SCIENCES, PERGAMON PRESS, OXFORD, GB, vol. 81, no. 13, 20 September 2007 (2007-09-20), pages 1071-1078, XP022263014 ISSN: 0024-3205

## Description

The invention relates to novel compositions comprising magnolol and honokiol wherein the mol ratio of magnolol to honokiol is between 0.2 and 0.4 as well as to the use of these compositions as a medicament, in particular as a medicament for the treatment, co-treatment or prevention of inflammatory disorders.

Inflammatory disorders are one of the most important health problems in the world. Inflammation is in general a localized protective response of the body tissues to invasion of the host by foreign material or injurious stimuli. The causes of inflammation can be infectious agents such as bacteria, viruses, and parasites; or physical agents such as bums or radiation; or chemicals like toxins, drugs or industrial agents; or immunological reactions such as allergies and autoimmune responses or conditions associated with oxidative stress.

Inflammation is characterized by pain, redness, swelling, heat, and eventual loss of function of the affected area. These symptoms are the results of a complex series of interactions taking place between the cells of the immune system. The response of the cells results in an interacting network of several groups of inflammatory mediators: Proteins (e.g. cytokines, enzymes (e.g. proteases, peroxydase), major basic protein, adhesion molecules (ICAM, VCAM), lipid mediators (e.g. eicosanoids, prostaglandins, leukotrienes, platelet activating factor (PAF)), reactive oxygen species (e.g. hydroperoxides, superoxyde anion O₂⁻, nitric oxide (NO) etc). However, many of those mediators of inflammation are also regulators of normal cellular activity. Thus, deficiencies of inflammatory reactions lead to a compromised host (i.e. infection) while uncontrolled and thus chronic inflammation leads to inflammatory diseases mediated in part by the excessive production of several of the above mentioned mediators.

Acute and chronic inflammation resulting from an excessive biosynthesis of inflammatory mediators is involved in numerous inflammatory disorders such as arthritis (*e*.*g*. osteoarthritis, rheumatoid arthritis), asthma, inflammatory bowel diseases, inflammatory diseases of the skin and chronic inflammatory disorders, such as atherosclerosis, heart diseases, metabolic syndrome X, osteoporosis, cancer, Alzheimer's disease and pre-stages thereof such as mild cognitive impairment.

Arthritis is a chronic (inflammatory) disease of the joints and encompasses many different forms. For example, arthritis includes rheumatoid arthritis, spondyloarthopathies, gouty arthritis, osteoarthritis, systemic lupus erythematosus and juvenile arthritis. Like asthma, rheumatoid arthritis is characterized at the molecular level by chronically unbalanced expression of cytokines, chemokines, kinins and their receptors, adhesion molecules and their respective receptors, as well as inflammatory enzymes.

Currently, two main classes of drugs, the corticosteroid and the nonsteroidal anti-inflammatory drugs (NSAIDs) are used to treat inflammatory disorders. NSAIDs and corticosteroids provide essentially symptomatic relief. Use of corticosteroids has declined due to a growing concern about the serious side effects of prolonged use. Long-term use of NSAIDs when treating chronic diseases such as arthritis, is limited by severe side-effects like serious gastrointestinal complications, renal toxicity or asthmatic reactions.

Therefore, there is a need for new anti-inflammatory agents with weak or no side effects. Patients with inflammatory diseases have a special interest in a type of treatment considered as "natural" with mild anti-inflammatory effects and without major side effects, which can be used for disease prevention and as adjuvant treatment. Furthermore, the treatment used needs to maintain the equilibrium between excessive and insufficient inflammatory reaction.

There are many known examples of such "natural" agents with shown anti-inflammatory action. However, a disadvantage of these "natural" compounds is that their biological and thus inhibitory activity is often inadequate.

Thus, it was an object of the present invention to provide nature derived active ingredients that are effective in the treatment of inflammatory disorders without having major side effects.

Magnolol and Honokiol as well as Magnolia Bark extracts have been described to have an anti-inflammatory activity. However, it has now surprisingly been found that a specific mol ratio of magnolol (MA) and honokiol (HO) shows a synergistically enhanced anti-inflammatory activity on inflammatory markers such as prostaglandin PGE₂ and nitric oxide. Furthermore, it has surprisingly been found, that this specific mol ratio also synergistically enhances cartilage build-up and has a synergistically enhanced chondroprotective effect.

Thus, the invention relates to compositions comprising magnolol and honokiol, wherein the mol ratio of magnolol to honokiol is in the range of 0.2 to 0.4, preferably in the range of 0.22 to 0.40, most preferably of 0.23 to 0.4, in particular of about 0.25 to 0.35 such as e.g. 0.28 to 0.33, in particular about 0.33.

In a preferred embodiment, the invention relates to a composition according to the invention for the treatment, co-treatment or prevention of inflammatory disorders. Exemplary inflammatory disorders encompass heart disease, multiple sclerosis, osteo- and rheumatoid arthritis, atherosclerosis, and osteoporosis without being limited thereto.

As the specific mol ratio of magnolol and honokiol next to its anti-inflammatory activity also synergistically enhances cartilage build up, the compositions according to the invention are especially suitable for the treatment, co-treatment and prevention of joint disorders, in particular arthritis, most in particular osteoarthritis and rheumatoid arthritis. Furthermore, the compositions are also suitable for inducing or enhancing cartilage repair or cartilage regeneration and are thus in particular attractive for regeneration and repair of cartilage injuries in joints such as for example traumatic cartilage injuries, degenerative joint disorders or sport injuries. Furthermore, the compositions of the present invention are suitable as an agent for treatment, co-treatment and prevention of joint disorders in particular for reduction of joint inflammation, maintenance and/or improvement of joint health, prevention of joint stiffness, increase of joint mobility, providing supple and/or flexible joints, lubrication of the joints, relief of pain associated with joint inflammation, decrease of joint swelling, lessening joint problems, and providing joint care. Furthermore, the compositions are suitable for the regeneration and/or maintenance of (articular) cartilage.

In another embodiment, the invention relates to the use of a composition according to the invention as a medicament, in particular for the treatment, co-treatment or prevention of inflammatory disorders with all the preferences as outlined above.

In a further embodiment, the invention relates to the use of a composition according to the invention as an agent for the treatment, co-treatment or prevention of inflammatory disorders. In one preferred embodiment the inflammatory disorder is a joint disorder in particular arthritis, most in particular osteoarthritis and rheumatoid arthritis.

Also, the invention relates to a method for treatment, co-treatment and prevention of inflammatory disorders with all the preferences as given above in animals including humans said method comprising the step of administering an effective amount of a composition according to the invention.

The term 'effective amount' as used herein refers to an amount necessary to obtain a physiological effect. The physiological effect may be achieved by one single dose or by repeated doses. The dosage administered may, of course, vary depending upon known factors, such as the physiological characteristics of the particular composition and its mode and route of administration; the age, health and weight of the recipient; the nature and extent of the symptoms; the kind of concurrent treatment; the frequency of treatment; and the effect desired and can be adjusted by a person skilled in the art.

The magnolol and honokiol used according to the invention may be obtained either by chemical synthesis or by isolation from plant material such as e.g. from *Magnolia officinalis or Magnolia grandifloris.* Thus, the term 'magnolol' and honokiol' encompasses both "natural" (isolated) and "synthetic" (manufactured) magnolol and honokiol.
Magnolol is also known as 5,5'-Diallyl-2,2'-biphenyldiol, respectively 5,5'-di-2-propenyl-[1,1'-Biphenyl]-2,2'-diol (CAS [528-43-8]) and honokiol as 3',5-Diallyl-2,4'-biphenyldiol, respectively 3',5-di-2-propenyl-[1,1'-Biphenyl]-2,4'-diol (CAS [35354-74-6]).

In the framework of the invention, with animals is meant all animals, including mammals, examples of which include humans. Preferred examples of mammals beside humans are non-ruminant or ruminant animals including cats, dogs, dromedaries, camels, elephants, and horses.

The compositions according to the invention may be 'oral compositions' as well as 'topical compositions'.

The term 'oral composition' as used herein denotes compositions that are administered orally. Thus, oral compositions according to the present invention can serve as supplements to food, feed and beverages, as dietary supplements or as pharmaceutical preparations which may be solid - such as powders, capsules or tablets - or liquid - such as solutions or suspensions. Furthermore the term 'oral composition' also comprises food, feed and beverages containing magnolol and honokiol in the mol ratio and with the preferences as given above. The term food also include food products or foodstuffs such as e.g. functional foods and prepared food products, the latter referring to any pre-packaged food approved for human consumption.

The term 'topical composition' as used herein refers to any cosmetic or pharmaceutical composition that can be topically applied to mammalian keratinous tissue. The term 'cosmetic composition' as used in the present application refers to cosmetic compositions as e.g. defined under the heading "Kosmetika" in Römpp Lexikon Chemie, 10th edition 1997, Georg Thieme Verlag Stuttgart, New York.

Examples of other application forms for compositions according to the invention include parenteral administration or administration via suppositories without being limited thereto.

The dosage of 'magnolol and honokiol in the mol ratio and with the preferences as given above' via an oral composition will, of course, vary depending upon known factors, such as the physiological characteristics of the particular composition and its mode and route of administration; the age, health and weight of the recipient; the nature and extent of the symptoms; the kind of concurrent treatment; the frequency of treatment; and the effect desired which can be determined by the expert in the field with normal trials, or with the usual considerations regarding the formulation of a oral composition.

In a preferred embodiment, the oral compositions such as e.g. a food, feed or beverage comprises per serving an amount of 0.01 to 1g, more preferably 0.2 mg to 500 mg of magnolol and honokiol with the mol ratio and preferences as given above.

In another embodiment the oral composition such as e.g. a dietary supplements or a pharmaceutical preparation may comprise the magnolol and honokiol in the mol ratio and with the preferences as given above in an amount of preferably 1 mg to 2000 mg per dosage unit, *e.g*., per capsule or tablet, or from 1 mg per daily dose to 3000 mg per daily dose of a liquid formulation. In a preferred embodiment the magnolol and honokiol in the ratio and with the preferences as given above are administered via a pharmaceutical composition either in the form of a single dose or by multiple doses in an amount of at least 0.01 mg/ kg bodyweight/ day, preferably in an amount of 0.1-50 mg/ kg body weight/ day, most preferably in an amount of 0.3-15 mg/ kg body weight / day.

The oral compositions according to the present invention may be in any galenic form that is suitable for administering to the animal body including the human body, more in particular in any form that is conventional for oral administration, e.g. in solid form, for example as (additives/supplements for) food or feed, food or feed premixes, fortified food or feed, tablets, pills, granules, dragées, capsules, and effervescent formulations such as powders and tablets, or in liquid form, for instance in the form of solutions, emulsions or suspensions, for example as beverages, pastes and oily suspensions. The pastes may be filled into hard or soft shell capsules. The compositions according to the invention may also be in the form of controlled (delayed) release formulations.

If the composition is prepared in form of tablets, capsules, granules or powder for oral administration, there may be used excipients such as lactose, sucrose, sodium chloride, glucose, urea, starch, dextrins and/or maltodextrins, calcium carbonate, calcium phosphate and/or calcium hydrogen phosphate, kaolin, crystalline and/or microcrystalline cellulose and/or silicic acid as carriers; binders such as water, ethanol, propanol, simple syrup, glucose solution, starch and/or hydrolyzed starch solution, gelatin solution, carboxymethylcellulose, hydroxypropylcellulose, hydroxypropylstarch, shellac, methylcellulose, ethylcellulose, calcium phosphate and/or polyvinyl pyrrolidone; disintegrators such as dry starch, croscarmellose, crospovidone, sodium alginate, agar powder, laminaran powder, sodium hydrogencarbonate, calcium carbonate, polyoxyethylene sorbitan fatty acid esters, sodium lauryl sulfate, stearic acid monoglyceride, starch and/or lactose; disintegration-preventing agents such as stearic acid, cacao butter and/or hydrogenated oils; absorbefacients such as quaternary ammonium bases and/or sodium lauryl sulfate; humectants such as glycerol and/or starch; adsorbents such as starch, lactose, kaolin, bentonite and/or colloidal silica; lubricants such as purified talc, stearic acid salts, boric acid powder and/or polyethylene glycol; taste corrigents such as sucrose, orange peel, citric acid and/or succinic acid; and the like.

If the composition is prepared in the form of tablets, these may be provided as tablets coated with usual coatings, for example, sugar-coated tablets, gelatin-coated tablets, enteric coated tablets, film-coated tablets, double coated tablets, multilayer-coated tablets and the like. The capsules are prepared by mixing the compounds according to the present invention with the various carriers exemplified above or according to the current state of the art and charging the mixture into hard gelatin capsules, soft capsules and the like.

A multi-vitamin and mineral supplement may be added to the compositions according to the present invention, e.g. to maintain a good balanced nutrition or to obtain an adequate amount of an essential nutrient missing in some diets. The multi-vitamin and mineral supplement may also be useful for disease prevention and protection against nutritional losses and deficiencies due to lifestyle patterns and common inadequate dietary patterns sometimes observed in diabetes. Suitable dosages for vitamins and minerals may be obtained, for example, by consulting the U.S. RDA guidelines.

Details on techniques for formulation of pharmaceutical preparations as well as on their administration are well known to a person skilled in the art and may be found e.g. in the latest edition of Remington's Pharmaceutical Sciences (Maack Publishing Co., Easton, PA) without being limited thereto. Additives and excipients for other oral compositions such as e.g. food additives are known to a person skilled in the art and are e.g. disclosed in Grundzüge der Lebensmitteltechnik by Horst-Dieter Tscheuschner, Behr (2008) or CRC Handbook of Food, Drug, and Cosmetic Excipients by Susan C. Smolinske et al, CRC Press Inc (1992).

Preferably, the topical preparations comprise magnolol and honokiol in the mol ratio and with the preferences as given above in an amount of at least 0.0001 wt.-%, preferably between 0.001 wt.-% and 20 wt.-%, more preferably between 0.01 and 10 wt.-%, still more preferably between 0.05 and 5 wt.-% such as about 0.1 to 1 wt.-%.

Regarding the kind of the topical preparation and the manufacture of the topical preparations as well as for further suitable additives, it can be referred to the pertinent literature, e.g. to Novak G.A., Die kosmetischen Präparate - Band 2, Die kosmetischen Präparate - Rezeptur, Rohstoffe, wissenschaftliche Grundlagen (Verlag für Chem. Industrie H. Ziolkowski KG, Augsburg).

The topical compositions according to the invention may be in the form of a suspension or dispersion in solvents or fatty substances, or alternatively in the form of an emulsion or micro emulsion such as e.g. liquid or solid oil-in-water emulsions, water-in-oil emulsions, multiple emulsions (e.g. O/W/O or W/O/W-type emulsions), microemulsions, PIT-emulsions, pickering emulsions. Furthermore, the compositions may be in the form of e.g. a cream, a paste, a lotion, a thickened lotion, a hydrogel, an alcoholic gel, a lipogel, a one or multiphase solution, or a milk, a vesicular dispersion in the form of an ointment, a gel, a solid tube stick or an aerosol mousse. If desirable, the topical compositions according to the invention may be provided in the form of a mousse, foam or a spray foams, sprays, sticks or aerosols or wipes.

The topical compositions of the invention may further comprise the usual cosmetic respectively dermatological adjuvants and/or additives such as preservatives/ antioxidants, fatty substances/ oils, water, organic solvents, silicones, thickeners, softeners, emulsifiers, additional light screening agents, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, light stabilizers, insect repellants, skin tanning agents, skin whitening agents, antibacterial agents, preservatives active ingredients or any other ingredients usually formulated into cosmetics. The necessary amounts of the cosmetic and dermatological adjuvants, additives and/or additional active ingredients can, based on the desired product, easily be chosen by a person skilled in the art.

The amount of the topical composition which is to be applied to the skin depends on the concentration of the active ingredients in the preparation and the desired cosmetic or pharmaceutical effect. For example, the application can be such that a crème is applied to the skin. A crème is usually applied in an amount of about 1 to 2 mg crème/cm² skin. The amount of the composition which is applied to the skin is, however, not critical, and if with a certain amount of applied composition the desired effect cannot be achieved, a higher concentration of the active preparations which contain more active ingredient might be employed.

Surprisingly it has been found that the anti-inflammatory as well as the cartilage build-up and repair activity of the compositions according to the invention can be further synergistically enhanced by the addition of an effective amount of glucosamine or chrondroitin.

Thus, the invention furthermore relates to compositions according to the invention further comprising an effective amount of glucosamine as well as to compositions according to the invention further comprising an effective amount of chondroitin and to compositions according to the invention further comprising an effective amount of glucosamine and chondroitin. Of course, the invention also relates to the use of such compositions according to the invention as outlined above

In the framework of the present invention, with glucosamine is meant glucosamine and all derivatives thereof such as glucosamine salts, for instance glucosamine sulfate or glucosamine hydrochloride. Glucosamine may be prepared from shell chitin, which is typically sourced from crab or shrimp. Glucosamine is commercially available and its daily intake by a human adult (weighting approximately 70kg) is preferably between 100 and 2000 mg per day. An oral composition according to the invention preferably comprises 5 mg to 1000 mg of glucosamine per serving. A pharmaceutical may preferably comprise glucosamine in an amount from 10 mg to 1000 mg per dosage unit, e.g., per capsule or tablet, or from 500 mg per daily dose to 2000 mg per daily dose of a liquid formulation. If the composition is a topical composition the amount of glucosamine may be selected in the range 0.001 wt.-% and 20 wt.-%, more preferably between 0.01 and 10 wt.-%, still more preferably between 0.05 and 5 wt.-% such as about 0.1 to 1 wt.-%.

The ratio (w/w) of magnolol and honokiol to glucosamine in the compositions according to the invention may be selected in the range of 1 to 50 to 5 to 1, preferably in the range of 1 to 20 to 3 to I such as e.g. in the range of 1 to 10 to 1 to 1.

In the framework of the present invention, with chondroitin is meant a sulfated glycosaminoglycan (GAG) composed of repeating disaccharide subunits. The source material for chondroitin may be bovine cartilage. The chains of the disaccharides vary in length from 20 to 80. Daily intake of chondroitin by a human adult (weighing approximately 70kg) is preferably between 100 and 2000 mg, more preferably between 800 to 1200 mg per day. An oral composition preferably comprises 5 mg to 1000 mg of chondroitin per serving. A pharmaceutical may preferably comprise chondroitin in an amount from 10 mg to 1000 mg per dosage unit, *e*.*g*., per capsule or tablet, or from 500 mg per daily dose to 2000 mg per daily dose of a liquid formulation. A topical composition preferably comprises an amount of chondroitin in the range 0.001 wt.-% and 20 wt.-%, more preferably between 0.01 and 10 wt.-%, still more preferably between 0.05 and 5 wt.-% such as about 0.1 to 1 wt.-%.

The ratio (w/w) of magnolol and honokiol to chondroitin in the compositions according to the invention may be selected in the range of 1 to 50 to 50 to 1, preferably in the range of 1 to 25 to 25 to 1, such as e.g. in the range of 1 to 10 to 1 to 1.

The invention will now be elucidated by way of the following examples, without however being limited thereto.

### Brief description of the figures

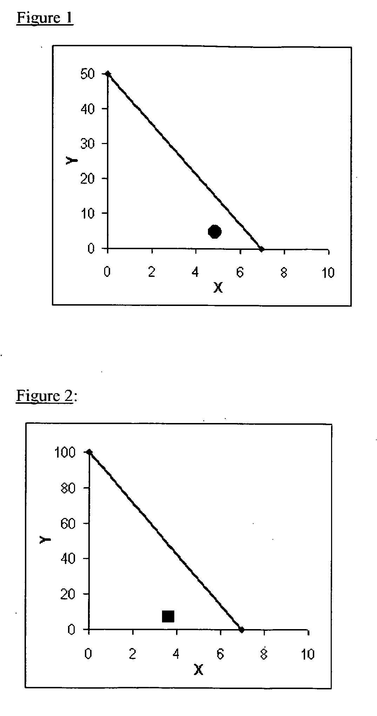

Figure 1: Synergistic effects of a mixture of MA/HO (1:3) and chondroitin sulfate on the inhibition of the inflammatory mediator nitric oxide (NO).
   The x-axis indicates the concentration (in µmol/L) of MA/HO (1:3) and the y-axis indicates the concentrations (in mg/L) of chondroitin sulfate. The end points of the straight line reflect the IC₅₀ values of chondroitin sulfate (y-axis) and of MA/HO (1:3 w/w) (x-axis). The observed IC₅₀ value of the combination of chondroitin sulfate and MA/HO (1:3 w/w) is plotted by the circular symbol. It lies below the straight line and thus mirrors synergistic interactions.
Figure 2: Synergistic effects of a mixture of MA/HO (1:3) and glucosamine sulfate on the inhibition of the inflammatory mediator nitric oxide (NO).
   Concentrations of MA/HO (1:3 w/w) are indicated on the x-axis (in µmol/L) and concentrations of glucosamine sulfate are indicated on the y-axis (in µmol/L). The end points of the straight line reflect the IC₅₀ values of glucosamine (y-axis) and of MA/HO (1:3 w/w) (x-axis). The observed IC₅₀ value of the combination of glucosamine and MA/HO (1:3 w/w) is plotted by the square symbol. It lies below the straight line and thus mirrors synergistic interactions.

The invention will now be elucidated by way of the following examples, without however being limited thereto.

### Examples

Honokiol (HO) and magnolol (MA) were from Honsea, Guangzhou, P.R. China, glucosamine sulphate and chondroitin sulphate (from shark) was purchased from Sigma.

### 1. Anti-inflammatory activity

The anti-inflammatory effects were determined in cellular assays by measuring the inhibition of the synthesis of nitric oxide and/or pro-inflammatory prostaglandins (PGE₂). PGE₂ plays a critical role in the inflammation process, while nitric oxide (NO) is a hallmark of inflammation in various chronic inflammatory diseases including various forms of arthritis, gastro-intestinal diseases and metabolic syndrome X. The effects on the inflammatory response were tested in cellular assays using a murine macrophage cell line, RAW264.7. The cells were purchased from ATCC (Manassas, VA, USA) and cultured in DMEM containing streptomycin/penicillin, non-essential amino acids and 10% fetal calf serum (FCS). In order to test a large range of concentration of MA and HO as well as mixtures thereof, cells (∼50'000/well) were seeded into flat-bottomed microtiter plates and cultured for one day. Cells were then starved in complete medium containing 0.25% FCS (D-025). After overnight culture, medium was removed and replaced by 100 µL of D-025 containing the test compounds at twice the final concentration. Subsequently, 100 µL of D-025 containing 2 µg/ml LPS was added (*i*.*e*. final LPS concentration of 1 µg/ml) and the cells cultured for 24 hours. Substances were usually tested in a concentration range from 0.2 to 50 µM in twofold dilution steps. All treatments were done in duplicates and several experimental series were done for each treatment. Concentrations of nitrite which was rapidly formed from nitric oxide released by cells were determined by the Griess reaction using sodium nitrite as standard. Briefly, 50 µl of supernatant was mixed with Griess reagent 1 (25 µL) and Griess reagent 2 (25 µL), centrifuged and the optical density at 540 nm determined. PGE₂ secreted into the cell culture medium was determined by EIA obtained from Cayman Chemicals (Ann Harbor, WI, USA) and used according to the manufacturer's instructions. All determinations were done in duplicates and at various dilutions of the culture supernatant. IC₅₀ values for LPS-stimulated cells were calculated using a two-parametric least-square fitting equation [y = A+((B-A) / (1+((C-x)^D))] for best-fit curves (Excel fit software program).

### 1.1. Determination of the optimal concentration range of MA to HO for the inhibition of the production of the inflammatory mediator NO

In order to determine the synergistic concentration range of MA to HO, various mixtures thereof were tested for their anti-inflammatory by measuring the inhibition of the synthesis of nitric oxide (NO) as outlined above. As can be seen from table 1, the optimal molar ratio of MA to HO lies within a range of about 0.3, (respectively about 1:3 w/w).

**Table 1.1:Inflammatory mediator NO**

| *Ratio MA to HO* | | IC₅₀ | | *Type of interaction* |
|---|---|---|---|---|
| *Wt.-%* | *Mol ratio* | *Observed* | *Expected ¹⁾* | |
| 0:100 | - | 4.58 | Reference HO | - |
| 10:90 | 0.11 | 5.27 | 5.01 | Non-synergistic |
| **22:78** | **0.28** | **4.50** | **5.53 ²⁾** | **Synergistic** |
| 40:60 | 0.67 | 8.31 | 6.38 | Non-synergistic |
| 60:40 | 1.5 | 8.93 | 7.23 | Non-synergistic |
| 90:10 | 9.0 | 8.54 | 8.48 | Non-synergistic |
| 100:0 | - | 8.91 | Reference MA | - |

| | | | | |
|---|---|---|---|---|
| ¹⁾ calculated as: [(IC₅₀ HO * ratio HO) + (IC₅₀ MA * ratio MA)]. ²⁾ An IC₅₀ value lower than the expected one reflects a synergistic interaction. | | | | |

### 1.2 Effect of a mixture of MA and HO on the production of the inflammatory mediator PEG₂

In order to elaborate, if the optimal concentration range determined in 1.1 also acts synergistically on other anti-inflammatory markers, the inhibition of the synthesis of PGE₂ by a mixture of MA/ HO in a molar ratio of about 0.3 has been determined. As shown in table 2, a mixture of MA/HO at a molar ratio of 0.31 also acts synergistically on the inhibition of PGE₂.

**Table 1.2: Inflammatory mediator PGE₂**

| *Ratio MA to HO* | | IC₅₀ | | *Type of interaction* |
|---|---|---|---|---|
| *Wt.-%* | *Mol ratio* | *Observed* | *Expected ¹⁾* | |
| 0:100 | - | 2.05 | Reference HO | - |
| 24:76 | 0.31 | **1.81***²⁾* | 2.78 | Synergistic |
| 100:0 | - | 5.09 | Reference MA | - |

| | | | | |
|---|---|---|---|---|
| ¹⁾ calculated as: [(IC₅₀ MA * ratio MA) + (IC₅₀ HO * ratio HO]. ²⁾ An IC₅₀ value lower than the expected one reflects a synergistic interaction. | | | | |

### 1.3 Effect of a mixture of MA and HO on the expression of inflammatory genes

Inflammatory responses are orchestrated by the transient activation of a variety of genes. Their regulation is tightly controlled *via* signaling pathways and eventually proteins that control the expression of genes such as transcription factors. Consequently, the effect of the compounds (each of them and various ratios of mixtures) has also been evaluated at the level of the expression of genes that are involved in the inflammatory response. These comprise genes of the prostaglandin synthesis pathway (*e.g*. COX-2), interleukins (*e*.*g*. IL1-α, IL-1β, IL-6), cytokines (*e*.*g*. TNF-α), inducible nitric oxide synthase (iNOS) and chemokines. RAW 264.7 cells were stimulated in the presence of different concentrations of substances. After 4 hours, RNA was extracted and the expression of genes determined by quantitative RT-PCR as described by Richard, N., Porath, D., Radspieler, A. and Schwager, J. in Mol Nutr Food Res 2005. 49: 431-442.

In table 3, the effect of MA and HO alone as well as of mixtures thereof in the indicated molar ratios on the expression of inflammatory genes or on anti-inflammatory transcription factors (I-κBα) is shown. The level of mRNA was determined by RT-PCR and expressed relative to the level observed in LPS-stimulated cells (*i*.*e*. without compounds). As can be retrieved from table 1.3, a combination of magnolol and honokiol at the indicated ratios exhibits a synergistic effect on the gene expression compared to the pure compounds which reflects a synergistically enhanced anti-inflammatory activity.

**Table 1.3:**

| *Gene* | *Expression level (%)* | | *Ratio MA:HO* | | *Expression level (%)* |
|---|---|---|---|---|---|
| | *(at 12.5* µ*mol*/*L)* | | | | *(at 12.5* µ*mol*/*L)* |
| | *MA* | *HO* | Wt.-% | Mol ratio | *Mixture MA*/*HO* |
| TNF-alpha | 96 | 81 | 20:80 | 0.25 | 70 ¹⁾ |
| IL-1alpha | 70 | 68 | 24:76 | 0.32 | 49 ¹⁾ |
| IL-10 | 136 | 94 | 24:76 | 0.32 | 67 ¹⁾ |
| I-κBalpha | 99 | 96 | 24:76 | 0.32 | 144 ²⁾ |

| | | | | | |
|---|---|---|---|---|---|
| ¹⁾ For pro-inflammatory genes (i.e. TNF-a, IL-1a, IL-10), a decreased expression level (compared to control which is set at 100%) reflects anti-inflammatory activity. ²⁾ For I-κBalpha an increased expression level (compared to control which is set at 100%) reflects anti-inflammatory activity. | | | | | |

### Example 1.4: Effect of a mixture of MA/HO (1:3) and glucosamine sulfate or chondroitin sulfate

The anti-inflammatory effect of a mixture of MA/HO (1:3 w/w) in combination with either glucosamine sulfate or chondroitin sulfate was evaluated as outlined above.

As can bee seen in table 1.4, glucosamine sulfate or chondroitin sulfate alone only show a marginal anti-inflammatory whereas the mixture of MA/HO (1:3 w/w) already itself exhibits an anti-inflammatory activity.

**Table 1.4: IC₅₀ values for single substances**

| *Substance* | *IC₅₀ Nitric Oxide* |
|---|---|
| Chondroitin sulfate | >50 mg/L |
| Glucosamine sulfate | >100 µmol/L |
| MA/HO (1:3 w/w) | 7.0 µmol/L |

The synergistic effect of a combination of a mixture of MA/HO (1:3 w/w) with glucosamine sulfate (ratio MA/HO (1:3 w/w) to glucosamine sulfate: 50µmol/L to 50 µmol/L) or chondroitin sulfate (ratio MA/HO (1:3 w/w) to chondroitin sulfate: 13.3 mg/L (i.e.50µmol/L) to 500 mg/L) on the inhibition of the inflammatory mediator nitric oxide (NO) has been evaluated based on the method published by Chou and Talalay (A simple generalized equation for the analysis of multiple inhibitions of Michealis-Menten kinetic systems. J. Biol. Chem. 1977. 252: 6438-6442). The results arc visualized in an isobologram wherein synergistic interactions between substances are reflected by an experimental value that lies below the straight line (see also: Bitler et al., J Nutr 2005. 135: 1475-1479).

As can bee seen in Figures 1 and 2, both combinations further synergistically enhance the anti-inflammatory activity of the mixture of MA/HO (1:3 w/w).

### 2 Effects on chondrocytes (cartilage build-up)

In articular cartilage, the balance between anabolic (build-up) and catabolic (break down) events needs to be maintained in order to prevent hypertrophy and excessive degradation of extracellular matrix (ECM), respectively. The ECM is built up of collagen and proteoglycans that are the products of collagen genes, for example human collagen 1 or aggrecan genes which are active during anabolic events. A substance which increases expression of these genes or the respective proteins (i.e. collagen and aggrecan) favours cartilage regeneration and/or build-up.

Catabolic events are controlled by the expression of genes, *e*.*g.* those genes that encode matrix metalloproteinases (MMPs) that eventually break down collagen or proteoglycans (ADAMTS-4, - 5). Of the MMPs, MMP-1 and MMP-3 have a major role in breaking down the ECM in cartilage degradation. Some genes including TIMPS-1 (tissue-inhibitor of MMPs) have anti-catabolic effects and thus contribute to prevention of tissue erosion.

The effect of MA and HO, mixtures thereof as well as combinations with glucosamine sulfate and chondroitin sulfate on the expression of human aggrecan, collagen I, collagen II, or chondrocyte transcription factors (SOX-6, -9) was measured *in vitro* in normal human chondrocytes (derived from knee) (CC-2550; Cambrex, respectively). A control experiment without the compounds was done concomitantly to compare the expression of these genes (percentage expression of gene in controls was set to 100%). Normal human chondrocytes were cultured for 4 hours with MA, HO as well as with a mixture of MA/ HO at the indicated ratio (at 12.5 µmol/L). Furthermore, combinations of a MA/HO (1:3 w/w) mixture with glucosamine or chondroitin sulfate were tested. The level of mRNA was determined by RT-PCR (Richard et al. Mol. Nutr. Food Res. 49, 431-442, 2005) and expressed relative to the level observed in cells cultured without the substances. The given values indicate the level of gene expression (in % of unstimulated cells [set at 100%] for anabolic and anti-catabolic genes; in % of IL1β-treated cells [set at 100%], for catabolic genes).

### 2.1 Synergistic effect of the combination MA/ HO on chondrocytes

In table 2.1 the effect of MA and HO alone as well as of mixtures thereof in the indicated molar ratios on the expression of genes that are involved in cartilage build-up is shown. As can be retrieved from table 2.1, honokiol and - to lesser extent - magnolol favors cartilage build-up and also diminish IL-1 gene expression as well as the respective receptor in un- (i.e. non-IL1beta-) stimulated cells. When combined in the inventive ratios, these effects, however, are synergistically enhanced.

**Table 2.1:**

| *Type of gene* | *Gene* | *Expression level (%)* | | *MA to HO* | | *Expression level (%)* |
|---|---|---|---|---|---|---|
| | | *(at 12.5* µ*mol*/*L)* | | | | *(at 12.5* µ*mol*/*L)* |
| | | *MA* | *HO* | *Wt.-%* | *Mol ratio* | *Mixture MA*/*HO* |
| Anabolic | Collagen I | 78 | 100 | 24:76 | 0.32 | 116¹⁾ |
| Anabolic | SOX-6 | 94 | 130 | 22:78 | 0.32 | 147¹⁾ |
| Anabolic | SOX-9 | 110 | 124 | 22:78 | 0.32 | 128¹⁾ |
| Anti-catabolic | TIMP-1 | 96 | 106 | 22:78 | 0.32 | 125¹⁾ |
| Catabolic Receptor for Il-1 | IL-RI | 98 | 89 | 22:78 | 0.32 | 83²⁾ |

| | | | | | | |
|---|---|---|---|---|---|---|
| ¹⁾ For anabolic and anti-catabolic genes an increased expression level (compared to control which is set at 100%) reflects a cartilage-rebuilding activity ²⁾ For catabolic genes a decreased expression level reflects a chondroprotective effect. | | | | | | |

### 2.2 Synergistic effect of the combination MA/ HO (1:3) with chondroitin sulfate on chondrocytes

The mixture of MA/HO (1:3 w/w) was used at 12.5 µmol/L (=3.325 mg/L), while the chondroitin sulfate (CS) was tested at 500 mg/L. The results shown in Table 2.2 indicate that a mixture of MA/ HO (1:3 w/w) substantially increases the expression of anabolic genes such as aggrecan. Similar effects were elicited by chondroitin. Yet, when combined the observed increase in gene expression exceeded the expected value, reflecting a synergistic interaction. Similar features were observed for the anti-catabolic gene TIMPS-1. Conversely, the expression of MMP-2 and ADAMTS-5 was synergistically reduced.
Numbers indicate the level of gene expression (in % of unstimulated cells [set at 100%] for anabolic genes and anti-catabolic; in % of IL1β-treated cells, for catabolic genes). MA/HO (1:3 w/w) and chondroitin sulfate (CS) were used at 12.5 µmol/L (*i*.*e*. 3.325 mg/L) and at 500 mg/L, respectively.

**Table 2.2.**

| *Type of gene* | *Gene* | *MA*/*HO (1:3)* | CS | *MA*/*HO + CS* |
|---|---|---|---|---|
| | | *(3.325 mg*/*L)* | *(500 mg*/*L)* | *(3.325 mg*/*L + 500 mg*/*L)* |
| Anabolic | Aggrecan (stim., 4hrs) | 147 | 62 | **173** ¹⁾ |
| Anabolic | SOX-9 | 87 | 147 | **152** ¹⁾ |
| Anti-catabolic | TIMP-1 (unstim., 4d) | 173 | 106 | **220** ¹⁾ |
| Catabolic | MMP-2 (4d) | 93 | 93 | **70** ²⁾ |
| Catabolic | ADAMTS-5 (stim. 4hrs) | 100 | 107 | **75** ²⁾ |

| | | | | |
|---|---|---|---|---|
| ¹⁾ For anabolic and anti-catabolic genes an increased expression level (compared to control which is set at 100%) reflects a cartilage-rebuilding activity. ²⁾ For catabolic genes a decreased expression level reflects a chondroprotective effect. | | | | |

### 2.3 Synergistic effect of the combination MA/ HO (1:3) with glucosamine sulfate on chondrocytes

The mixture of MA/HO (1:3 w/w) was used at 12.5 µmol/L, while the glucosamine sulfate (GS) was tested at 50 µmol/L. The results shown in Table 2.3 indicate that a mixture of MA/ HO (1:3 w/w) substantially increases the expression of anabolic genes such as aggrecan. Similar effects were elicited by glucosamine. Yet, when combined the observed increase in gene expression exceeded the expected one reflecting synergism

**Table 2.3.**

| *Type of gene* | *Gene* | *MA*/*HO (1:3)* | *GS* | *MA*/*HO + GS* |
|---|---|---|---|---|
| | | *(*12.5µmol/L*)* | *(50* µmol/L*)* | *(*12.5µmol/L + *50*µmol/L*)* |
| Anabolic | Aggrecan (4hrs) | 110 | 81 | **128** ¹⁾ |
| Anabolic | Collagen 1 (4hrs) | 99 | 121 | **157** ¹⁾ |
| Anabolic | Collagen II (4hrs) | 122 | 154 | **163** ¹⁾ |
| Anti-catabolic | TIMP-1 (4hrs) | 111 | 110 | **141** ¹⁾ |
| Catabolic | MMP-9 (stim. 4hrs) | 70 | 114 | **59** ²⁾ |
| Catabolic | ADAMTS-4 (unstim. 4d) | 98 | 101 | **85** ²⁾ |

| | | | | |
|---|---|---|---|---|
| ¹⁾ For anabolic and anti-catabolic genes an increased expression level (compared to control which is set at 100%) reflects a cartilage-rebuilding activity. ²⁾ For catabolic genes a decreased expression level reflects a chondroprotective effect. | | | | |

## Claims

1. A compositions comprising magnolol and honokiol, wherein the mol ratio of magnolol to honokiol is in the range of 0.2 to 0.4.

2. A composition according to claim 1 wherein the mol ratio of magnolol to honokiol is in the range of 0.22 to 0.40.

3. A composition according to claim 1 wherein the mol ratio of magnolol to honokiol is in the range of 0.25 to 0.35.

4. A composition according to anyone of claims 1 to 3 for use in the treatment, co-treatment or prevention of inflammatory disorders.

5. A composition according to claim 4 wherein the inflammatory disorders is a joint disorder.

6. A composition according to claim 5, wherein the joint disorder is arthritis.

7. The composition according to any ones of claims 1 to 6, which is an oral composition.

8. The composition according to any ones of claims 1 to 6, which is a topical composition.

9. The composition according to any ones of claims 1 to 8 further comprising glucosamine.

10. The composition according to claim 9 wherein the weight ratio of magnolol and honokiol to glucosamine is in the range of 1 to 10 to 1 to 1.

11. The composition according to any ones of claims 1 to 8 further comprising chondroitin.

12. The composition according to claim 9 wherein the weight ratio of magnolol and honokiol to chondroitin is in the range of 1 to 10 to 1 to 1.

13. Use of a composition according to any one of claims 1 to 12 for the manufacture of a medicament for the treatment, co-treatment and prevention of inflammatory disorders in animals including humans.

14. Use of a composition as defined in any of claims 1 to 12 which is a food product, foodstuff, dietary supplement, nutritional supplement, or a supplement composition for a food product or a foodstuff.

## Patentansprüche

1. Zusammensetzung, umfassend Magnolol und Honokiol, wobei das molare Verhältnis von Magnolol zu Honokiol im Bereich von 0,2 bis 0,4 liegt.

2. Zusammensetzung gemäß Anspruch 1, wobei das molare Verhältnis von Magnolol zu Honokiol im Bereich von 0,22 bis 0,4 liegt.

3. Zusammensetzung gemäß Anspruch 1, wobei das molare Verhältnis von Magnolol zu Honokiol im Bereich von 0,25 bis 0,35 liegt.

4. Zusammensetzung gemäß einem der Ansprüche 1 bis 3 für die Verwendung bei der Behandlung, Mitbehandlung oder Vorbeugung von entzündlichen Störungen.

5. Zusammensetzung gemäß Anspruch 4, wobei die entzündliche Störung eine Gelenksstörung ist.

6. Zusammensetzung gemäß Anspruch 5, wobei die Gelenksstörung Arthritis ist.

7. Zusammensetzung gemäß einem der Ansprüche 1 bis 6, welche eine orale Zusammensetzung ist.

8. Zusammensetzung gemäß einem der Ansprüche 1 bis 6, welche eine topische Zusammensetzung ist.

9. Zusammensetzung gemäß einem der Ansprüche 1 bis 8, ferner umfassend Glucosamin.

10. Zusammensetzung gemäß Anspruch 9, wobei das Gewichtsverhältnis von Magnolol und Honokiol zu Glucosamin im Bereich von 1 zu 10 bis 1 zu 1 liegt.

11. Zusammensetzung gemäß einem der Ansprüche 1 bis 8, ferner umfassend Chondroitin.

12. Zusammensetzung gemäß Anspruch 9, wobei das Gewichtsverhältnis von Magnolol und Honokiol zu Chondroitin im Bereich von 1 zu 10 bis 1 zu 1 liegt.

13. Verwendung einer Zusammensetzung gemäß einem der Ansprüche 1 bis 12 für die Herstellung eines Medikaments für die Behandlung, Mitbehandlung und Vorbeugung von entzündlichen Störungen bei Tieren, einschließlich Menschen.

14. Verwendung einer Zusammensetzung gemäß einem der Ansprüche 1 bis 12, welche ein Nahrungsmittelprodukt, Nahrungsmittel, Nahrungsergänzungsmittel, Ernährungsergänzungsmittel oder eine ergänzende Zusammensetzung für ein Nahrungsmittelprodukt oder ein Nahrungsmittel ist.

## Revendications

1. Compositions comprenant le magnolol et l'honokiol, **caractérisées en ce que** le rapport molaire entre le magnolol et l'honokiol est dans la gamme de 0,2 à 0,4.

2. Composition selon la revendication 1, **caractérisée en ce que** le rapport molaire entre le magnolol et l'honokiol est dans la gamme de 0,22 à 0,40.

3. Composition selon la revendication 1, **caractérisée en ce que** le rapport molaire entre le magnolol et l'honokiol est dans la gamme de 0,25 à 0,35.

4. Composition selon l'une quelconque des revendications 1 à 3, destinée à être utilisée dans le traitement, le co-traitement ou la prévention de troubles inflammatoires.

5. Composition selon la revendication 4, **caractérisée en ce que** le trouble inflammatoire est un trouble articulaire.

6. Composition selon la revendication 5, **caractérisée en ce que** le trouble articulaire est l'arthrite.

7. Composition selon l'une quelconque des revendications 1 à 6, **caractérisée en ce qu'**il s'agit d'une composition orale.

8. Composition selon l'une quelconque des revendications 1 à 6, **caractérisée en ce qu'**il s'agit d'une composition topique.

9. Composition selon l'une quelconque des revendications 1 à 8, comprenant en outre la glucosamine.

10. Composition selon la revendication 9, **caractérisée en ce que** le rapport en poids entre le magnolol plus l'honokiol et la glucosamine est dans la gamme de 1 sur 10 à 1 sur 1.

11. Composition selon l'une quelconque des revendications 1 à 8, comprenant en outre la chondroitine.

12. Composition selon la revendication 9, **caractérisée en ce que** le rapport en poids entre le magnolol plus l'honokiol et la chondroitine est dans la gamme de 1 sur 10 à 1 sur 1.

13. Utilisation d'une composition selon l'une quelconque des revendications 1 à 12, pour la fabrication d'un médicament destiné au traitement, au co-traitement et à la prévention de troubles inflammatoires chez l'animal, y compris l'homme.

14. Utilisation d'une composition telle que définie dans l'une quelconque des revendications 1 à 12, qui est un produit alimentaire, un aliment, un complément alimentaire, un complément nutritionnel, ou une composition de complément pour un produit alimentaire ou un aliment.
